(19) Europäisches Patentamt
European Patent Office
Office européen des brevets

(11) Publication number: 0 147 660
B1

(12) **EUROPEAN PATENT SPECIFICATION**

(45) Date of publication of patent specification: **08.11.89**

(51) Int. Cl.[4]: **G 01 N 33/84,** G 01 N 31/22

(21) Application number: **84114486.8**

(22) Date of filing: **29.11.84**

(54) **Colorimetric measuring method for zinc.**

(30) Priority: **02.12.83 JP 228221/83**

(43) Date of publication of application:
**10.07.85 Bulletin 85/28**

(45) Publication of the grant of the patent:
**08.11.89 Bulletin 89/45**

(84) Designated Contracting States:
**AT BE CH DE FR GB IT LI LU NL SE**

(56) References cited:
**CHEMICAL ABSTRACTS, vol. 101, no. 17, 22nd October 1984, page 353, no. 147313s, Columbus, Ohio, US; & JP - A - 84 30061 (WAKO PURE CHEMICAL INDUSTRIES, LTD.) 17-02-1984**

(73) Proprietor: **WAKO PURE CHEMICAL INDUSTRIES, LTD.**
**10, Doshomachi-3-chome**
**Higashi-ku Osaka (JP)**

(72) Inventor: **Yamazato, Fujio**
**3-8-540, Minamisuna 2-chome**
**Koto-ku Tokyo (JP)**
Inventor: **Tokuda, Kuniaki**
**515-13, Oaza Matoba**
**Kawagoe-shi Saitama (JP)**

(74) Representative: **Dr. Elisabeth Jung Dr. Jürgen Schirdewahn Dipl.-Ing. Claus Gernhardt**
**P.O. Box 40 14 68 Clemensstrasse 30**
**D-8000 München 40 (DE)**

(56) References cited:
**CHEMICAL ABSTRACTS, vol. 90, no. 6, 5th February 1979, page 601, no. 47864h, Columbus, Ohio, US; M. FURUKAWA: "2-2-(5-chloropyridyl)azor-5-dimethylaminophenol as a new sensitive reagent for zinc", & NAGOYA KOGYO GIJTSU SHIKENSHO HOKOKU 1978, 27(7), 223-9**

**EP 0 147 660 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
CHEMICAL ABSTRACTS, vol. 99, no. 2, 11th July 1983, page 634, no. 15732h, Columbus, Ohio, US; K. OHSHITA et al.: "Synthesis of N-sulfoalkyl derivatives of 2-(2-pyridylazo)-5-aminophenol and the spectrophotometric determination of uranium(VI)", & ANAL. CHIM. ACTA 1983, 149, 269-79

CHEMICAL ABSTRACTS, vol. 97, no. 6, 9th August 1982, page 650, no. 48790n, Columbus, Ohio, US; S-H HUNG et al.: "Spectrophotometric determination of silver with 2-(3,5-dibromo-2-pyridylazo)-5-diethylaminophenol in the presence of anionic surfactant", & TALANTA 1982, 29(2), 85-8

CHEMICAL ABSTRACTS, vol. 87, no. 26, December 1977, page 181, no. 204734y, Columbus, Ohio, US; R.V. PAMMENTER et al.: "The elimination of nickel from zinc plant solutions using dimethylglyoxime", & PAP. TASMANIA CONF. 1977, 291-8

Anal Chim ACTA 1983, 1449, 269-79

**Description**

Background of the Invention

(1) Field of the Invention:

The present invention relates to a novel method for colorimetrically measuring zinc in the presence of Fe, Cu, Co or Ni.

(2) Description of the Prior Art:

Recently, the quantitative measurement of a small amount of a metal rises in importance with respect to environmental sanitation and biochemical diagnosis method. In particular, zinc is a metal present in a small amount in the living organisms and one of the indispensable metal elements necessary for the growth, and is widely distributed in the bodies of the living organisms. It has been lately reported that the concentration of zinc in serum decreases in positive acrodermatitis, taste impediment, SLE (systemic lupus erythematosus), that zinc deficiency symptoms are observed in the case of administration of a high calorie transfusion, and so on, and the necessity of the measurement of the zinc in serum has been felt in the diagnosis, treatment and prognosis of these diseases.

As the method of quantitatively measuring a small amount of zinc until today, the atomic absorption method, the flame photometric method, and the colorimetric measuring method are mainly used, and the colorimetrically measuring method is advantageous from the standpoint that a number of specimens to be examined can be promptly treated and no special analysis device is necessary.

As the colorimetric reagent, use may be mainly made of dithizone, zincon, PAN{1-(2-pyridylazo)-2-naphthol}, but any one of them is not necessarily complete from the viewpoint of specificity. Further, the cyano compound or a variety of masking agents are required to be used in combination or extraction with an organic solvent is necessary in order that the interference of iron, copper, cobalt and manganese may be removed.

Under these circumstances, the present inventors have noted the compounds disclosed in Japanese Laid-Open Patent Application No. 91,975/1982 which have been developed as a high sensitivity colorimetrically measuring reagents for a small amount of a metal and have a molecular absorption coefficient of $7.0 - 13.3 \times 10^{-4}$. Although these compounds are all useful as high sensitivity colorimetrically measuring reagents for the heavy metal, they have the deficiency that they are also sensitive for small amounts of a metal other than zinc, especially in the case of pyridylazoaminophenol compounds.

Therefore, metals other than the intended specific ones are required to be masked.

For this purpose, use has been generally made of citrates, condensed phosphates, sodium fluoride, nitrilotriacetic acid and 1-hydroxyethane-1,1-diphosphonic acid as the masking agents for iron; salicylaldoxime, 2-mercaptobenzothiazol, dithiocarboxysarcosine, and dithiocarboxy glycine as the masking agents for copper; and dithiocarboxysarcosine and 2-mercaptobenzothiazol as the masking agents for cobalt.

It is, however, very inconvenient that some of these masking agents (for instance, citric acid, condensed phosphoric acid) also mask the zinc to be determined thereby lowering the sensitivity of the method. Additionally substances containing a partial structure —S—C=S or/and a —SH-group, which are used as the masking agent for copper and cobalt, are conspicuously poor in stability after the preparation thereof, and these substances may be a coloring promotor for iron because they reduce trivalent iron to divalent iron.

From "Chemical Abstracts", Vol. 90, 1979, it was known that solutions of the compound 2[2-(5-chloropyridyl)azo]-5-dimethylaminophenol in various mixed solvents, like MeOH—$H_2O$ form a violet Zn-complex with zinc, which can be used for the colorimetric determination if zinc. It is, however, clearly stated in this publication that other metal ions, like $Cu^{2+}$, $Co^{2+}$ and $Ni^{2+}$ seriously interfere in this method.

In the Journal "Talanta", Vol. 29, pp. 85 to 88 (1982) there is described a spectrophotometric determination method of silver, using the compound 2-(3,5-dibromo-2-pyridylazo)-5-diethylamino phenol as complexing agent. It is, however, clearly stated that a color change from orange-yellow to a red product takes only place in the presence of Na-lauryl sulfate at pH 5 and that in the absence of this lauryl sulfate no color reaction takes place as indication of the presence of the silver ion. In order to avoid interference of other metal ions, ethylenediamine tetraacetic acid (EDTA) is used as a masking agent.

Summary of the Invention

It is therefore an object of the present invention to provide a method for colorimetrically measuring zinc using a masking agent for Fe, Cu, Co and Ni which is excellent in stability and specifically acts upon those specific metals.

It is another object of the present invention to provide a method for colorimetrically measuring zinc with a high sensitivity.

According to the present invention, the method for colorimetrically measuring zinc using as a coloring reagent an aqueous solution of a 2-pyridylazoaminophenol derivative represented by the general formula (I):

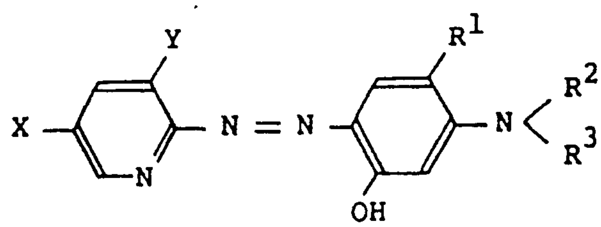

(I)

wherein X and Y represent a halogen atom or a hydrogen atom $R^1$ represents a hydrogen atom or a $C_{1-4}$-alkyl group, and $R^2$ and $R^3$ represent a hydrogen atom, a $C_{1-4}$-alkyl group, $-(CH_2)_nSO_3H$ in which n is an integer of 1—4,

$$-(CH_2)_l-\overset{\displaystyle OH}{\overset{|}{C}H}-(CH_2)_mSO_3H$$

in which l is an integer of 0—4 and m is an integer of 0—4 provides that at least one of $R^2$ and $R^3$ is $-(CH_2)_lSO_3H$ or

$$-(CH_2)_l-\overset{\displaystyle OH}{\overset{|}{C}H}-(CH_2)_mSO_3H$$

or a salt thereof, is characterized in that one or more kinds of surface active agents are used as a masking agent for Fe, Cu, Co and Ni.

These and other objects, features and advantages of the invention will be well appreciated upon reading of the following description.

Detailed Description of the Invention

The present inventors have made various thorough studies and examinations of a method of colorimetrically measuring zinc using as a coloring reagent an aqueous solution of a 2-pyridylazoaminophenol derivative represented by the following formula (I):

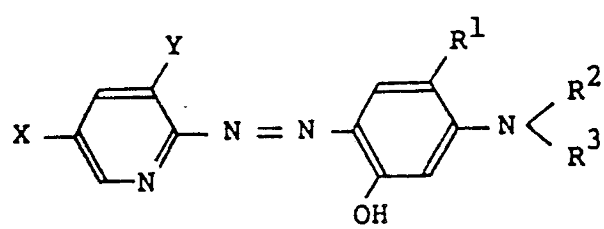

(I)

as defined above and found that a surface active agent exhibits a great effect as a masking agent for Fe, Cu, Co or Ni.

That is, the present invention relates to a method of colorimetrically measuring zinc by using an aqueous solution of a 2-pyridylazoaminophenol derivative represented by the general formula (I) or the salt thereof as a coloring reagent, wherein one or more kinds of surface active agents are used as a masking agent for the above specified metals.

The surface active agents are used in the colorimetrically analyzing method of the prior art as a solubilizing agent for coloring reagents hard to dissolve, and for the purpose of shifting of absorption wavelength and prevention of turbidity of the sample, clarification of the sample, stabilization of color development, isolation of the intended metal from the metal combined protein or extraction of produced chelate into an organic solvent. The present inventors have found that the surface active agents can be used for a purpose quite different from the above-mentioned ones, that is, they can act as the masking agent for the above-specified metals.

The surface active agents used in the present invention are not specifically restricted, and those that dissolve to a clear solution in a range of pH 4—10, which is appropriate for the coloring reagent, are preferred.

Particularly nonionic, cationic and amphoteric surface active agents are preferable because they do not interfere with the coloring of zinc, and inhibit the coloring or iron, copper, cobalt and nickel. Further similar effects can be obtained when the anionic surface active agent and the nonionic surface active agent are used in combination. When these surface active agents are used together with the conventional masking agent, the effects of the above-mentioned surface active agents are remarkably increased.

As the nonionic surface active agents particularly effective for the present invention, use may be made of Triton X—100 (polyoxyethylene iso-octylphenyl ether, Rohm & Haas Co., Ltd., trade name) Brij-35

(polyoxyethylene lauryl ether, Kao Atlas Co. Ltd., trade name), Emulgen 120 (polyoxyethylene lauryl ether, Kao Atlas Co. Ltd., trade name), Tween 20 (polyoxyethylensorbitan monolauryl ether, Kao Atlas Co. Ltd., trade name), Tween 80 (polyoxyethylene sorbitan monooleyl ether, Kao Atlas Co. Ltd., trade name), Softanol 90 (polyoxyethylene alkyl ether, Nippon Catalyst Chemical Industries Ltd.), Triton X—405 (polyoxyethylene, Rohm & Haas Co., Ltd., trade name), Emulgen 147 (polyoxyethylene lauryl ether, Kao Atlas Co., Ltd., trade name), Emulgen 920 (polyoxyethylene lauryl ether, Kao Atlas Co., Ltd., trade name), Emulgen 950 (polyoxyethylene nonylphenyl ether, Kao Atlas Co., Ltd., trade name), Emulgen PP 290 N(oxyethylene-oxypropylene block polymer, Kao Atlas Co., Ltd., trade name) and Emusol 3130 (polyoxyethylenesorbitanemonostearate, Kao Atlas Co., Ltd., trade name).

As to the anionic surface active agents, use may be made of Sundet EMN (polyethylenealkyl ether sulfuric acid ester sodium salt, Sanyo Kasei Kogyo Co., Ltd., trade name), Emarl NC (polyoxyethylenealkylphenyl ether sulfuric acid ester, Kao Atlas Co., Ltd., trade name), Aranon ACE (N-cocoyl-N-methyl-β-alanine sodium salt, Kawaken Fine Chemical Co., Ltd., trade name), Emarl 20 C (polyoxyethylene alkylsulfate sodium salt, Kao Atlas Co., Ltd., trade name), Nonipol S—40 (polyethylene alkylphenyl ether sulfuric acid ester sodium salt, Sanyo Kasei Kogyo Co., Ltd., trade name), sodium lauryl sulfate (sodium salt of lauryl sulfuric acid ester), Sundet BL (alkylbenzene sulfonic acid formalin condensate, Sanyo Kasei Kogyo Co., Ltd., trade name), Levenol WX (polyoxyethylenealkyl sulfate sodium salt, Kao Atlas Co., Ltd., trade name), Ultrafon W (pentadecylbenzimidazol sulfonic acid sodium salt, Nippon CIBA-GEIGY Co., Ltd., trade name), Demol N (naphthalene sulfonic acid formalin condensate, Kao Atlas Co., Ltd., trade name), Softanol 30S -25(secondary alkyl sulfuric acid sodium salt, Nippon Catalyst Chemical Industries, Ltd., trade name) and Levenol WZ (polyoxyethylenealkyl sulfate sodium salt, Kao Atlas Co., Ltd., trade name).

As the cationic surface active agents, use may be made of Cotamine 24P (lauryltrimethylammonium chloride, Kao Atlas Co., Ltd., trade name), Cotamine 86P (stearyltrimethylammonium chloride, Kao Atlas Co., Ltd., trade name) and Levasorp NL (laurylpyridinium bromide, Miyoshi Yushi Co., Ltd., trade name).

As the amphoteric surface active agents, use may be made of Anhitol 24B (laurylbetaine, Kao Atlas Co., Ltd., trade name) and Anhitol 86B (stearylbetaine, Kao Atlas Co., Ltd.).

Although the effective concentration of the surface active agent in the present invention is not specifically restricted, 0.01—20.0% is preferred.

The 2-pyridylazoaminophenol derivative of general formula (I) as defined above or the salt thereof used as a coloring reagent has a partial structure as a chromophore:

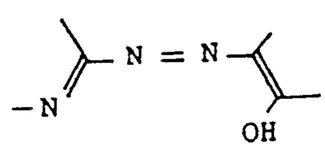

Typical compounds include for instance:

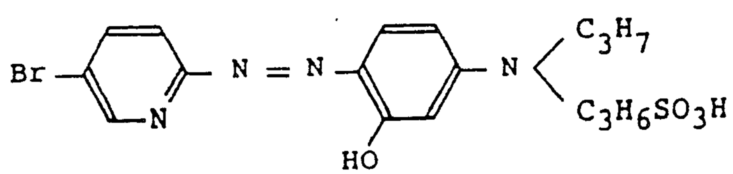

(1)

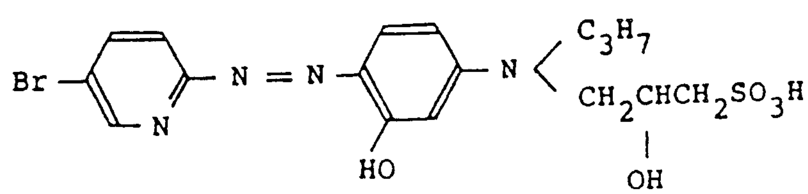

(2)

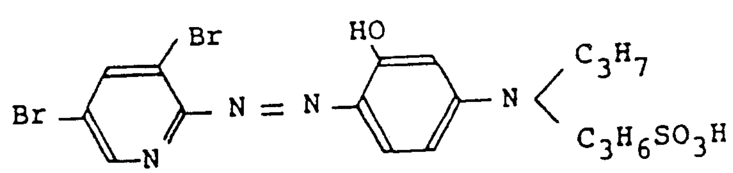

(3)

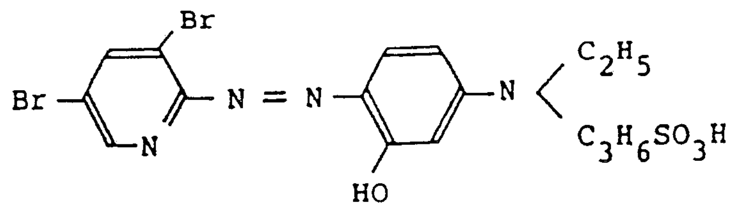

(4)

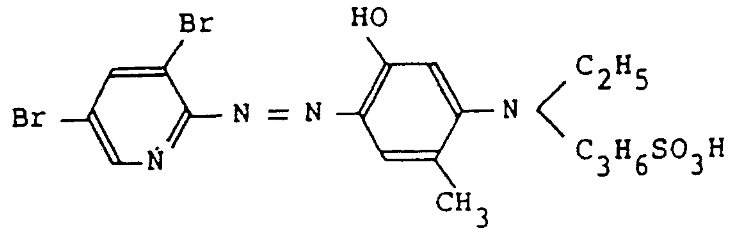

(5)

Among a series of compounds which are described as or similar to a group of compounds disclosed in Japanese Patent Application No. 91,975/1982, the compounds in which the ortho position to an azo group of a benzene ring is substituted by a carboxyl group or an amino group are not necessarily satisfactory for the method of the present invention, and the substituting group is a hydroxy group. In the case that X and Y of the general formula (I) are halogen atoms, the compounds are most easily synthesized when the halogen atoms are bromine atoms, and when they are chlorine, the synthesis is easier next to thé former case. The number of carbons in the alkyl groups of $R^1$, $R^2$ and $R^3$ is 1—4. The salt of the coloring reagent of the general formula (I) according to the present invention is an organic amine salt such as diethyl-amine salt or triethylamine salt, an inorganic salt such as ammonium salt, alkali metal salt or alkaline earth metal salt. Among the coloring reagents disclosed in Japanese Laid-Open Patent Application No. 91,975/1982, those compounds of the general formula (I) in which at least one of $R^2$ and $R^3$ has a sulfonic acid group are soluble in water. They are therefore suitable for use in the present invention.

The present inventors have further made thorough studies as regards the methods of microanalysis which are specific to zinc and are high in sensitivity, and as a result they have found that when dimethylglyoxime which has conventionally been used as a coloring reagent of nickel is used together with the coloring reagent of the invention and the surface active agent, dimethylglyoxime surprisingly becomes an effective masking agent for nickel and cobalt. Based on this finding, the present inventors have accomplished novel and useful embodiments of the present invention in which the compound which has not been used as the masking agent in the conventional analysis method is used as the masking agent for nickel and cobalt.

That is, the present invention relates also to a method of colorimetrically measuring zinc in which one or more kinds of the surface active agents are used together with 2-pyridylazoaminophenol derivative of formula (I) as defined above or the salt thereof as a coloring reagent, and dimethylglyoxime is used as an additional masking agent for nickel and/or cobalt.

Dimethylglyoxime used in the present invention which is a masking agent for nickel and/or cobalt is generally used in a free form or as disodium salt or in a mixed form thereof depending on the case, and may be used in a coloring reagent solution ordinarily at the concentration of 0.005—1.0%, preferably at the concentration of 0.01—0.5%.

On the other hand, appropriate use amounts of the masking agents of other metals are determined depending upon the masking effect, the influence thereof upon zinc, the solubility and so forth. For instance, when salicyaldoxime is used as the masking agent for copper, it is used ordinarily at the concentration of 0.005—0.5%, preferably at the concentration of 0.01—0.3%. When a citric acid salt is used as the masking agent iron, it is used ordinarily at the concentration of 1.0—8.0%, preferably at the concentration of 2.0—5.0%.

As specific examples of the surface active agents and 2-pyridylazoaminophenol derivative or the salt thereof used in the present invention, the compounds exemplified in the first aspect of the present invention are used preferably too. The addition amount of these compounds are the same as mentioned above.

According to the method of the present invention, when a sample is serum, protein is removed therefrom, and then the sample is added to a reagent of the present invention in which the respectively appropriate amounts of the surface active agent and the coloring reagent are dissolved into an aqueous buffer solution of a given pH, or a reagent of the second aspect of the present invention in which an appropriate amount of dimethylglyoxime is dissolved into the former reagent, and the thus obtained solution is left for a short time, for example, 5 or 10 minutes. Thereafter, absorbance is measured at 555 nm with reference to a reagent blank as control, and the concentration of zinc in the sample is determined from comparison with a calibration curve.

The method of the present invention will be explained below with reference to the following example.

Example 1

Reagents:

(1) Coloring reagent solution:

0.05 mM of each of five kinds of coloring reagents was dissolved into 600 ml of 0.05 M boric acid buffer solution of pH 8.5, to which Brij-35, sodium laurylbenzene sulfonate or Sundet ENM was added in an amount of 1.6%, and dilution was made with water to give 1 liter in a total volume.

(2) Sample solution A:

14 mg (5 mg when calculated as zinc) of zinc acetate was dissolved into water to be 1 liter in a total volume.

(3) Sample solution B:

48 mg (10 mg when calculated as iron) or ammonium iron (III) sulfate was dissolved into 0.1 N—HCl to be 100 ml in a total volume.

(4) Sample solution C:

18 mg (5 mg when calculated as copper) of copper sulfate was dissolved into water to be 100 ml in a total volume.

(5) Sample solution D:

11 mg (5 mg when calculated as nickel) of nickel chloride was dissolved into water to be 100 ml in a total volume.

(6) Sample solution E:

11 mg (5 mg when calculated as cobalt) of cobalt chloride was dissolved into water to be 100 ml in a total volume.

Test method:

3 ml of the coloring reagent solution was added to 20 µl of each of Sample solutions A—E, which was left at room temperature for 20 minutes. Thereafter, absorbance was measured at 555 nm with reference to a reagent blank as control.

In Table 1 are shown differences in coloring degree among the coloring reagents with the respective metal ions depending upon the presence or the absence of the surface active agents. The coloring degrees of the respective metals are changed by the addition of the surface active agents, and particularly the coloring degree of Fe is lowered to about one fourth when Brij-35 was added, thereby relatively enhancing the specificity of zinc.

Table 1

| Coloring reagent | Surface active agent | Zn | Cu II | Fe III | Ni II | Co III | |
|---|---|---|---|---|---|---|---|
| | | 5mg/dl | 5mg/dl | 10mg/dl | 5mg/dl | 5mg/dl | 555 nm OD |
| 5Br-PAPS 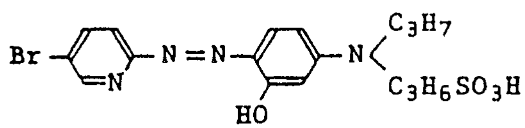 | No | 0.826 | 0.671 | 0.195 | 0.940 | 0.582 | Every figure shows the absorbance at 555 nm |
| | Brij-35 | 0.760 | 0.641 | 0.052 | 0.613 | 0.526 | |
| | SLS | 0.813 | 0.522 | 0.086 | 0.780 | 0.531 | |
| | Sundet ENM | 0.616 | 0.581 | 0.281 | 0.730 | 0.506 | |
| 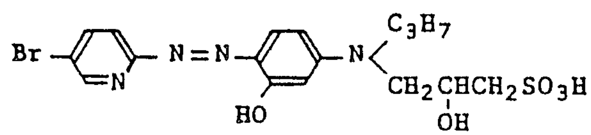 | No | 0.621 | 0.646 | 0.173 | 0.795 | 0.529 | |
| | Brij-35 | 0.571 | 0.616 | 0.046 | 0.518 | 0.478 | |
| | SLS | 0.614 | 0.502 | 0.076 | 0.660 | 0.482 | |
| | Sundet ENM | 0.459 | 0.559 | 0.249 | 0.617 | 0.460 | |
| 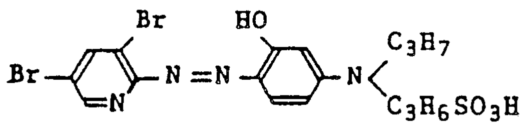 | No | 0.601 | 0.675 | 0.143 | 0.829 | 0.493 | |
| | Brij-35 | 0.563 | 0.622 | 0.038 | 0.541 | 0.445 | |
| | SLS | 0.592 | 0.532 | 0.063 | 0.688 | 0.450 | |
| | Sundet ENM | 0.432 | 0.509 | 0.201 | 0.641 | 0.429 | |
| 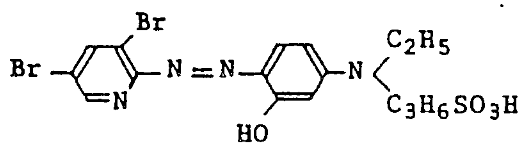 | No | 0.677 | 0.562 | 0.187 | 0.830 | 0.564 | |
| | Brij-35 | 0.613 | 0.537 | 0.049 | 0.540 | 0.510 | |
| | SLS | 0.666 | 0.437 | 0.083 | 0.688 | 0.515 | |
| | Sundet ENM | 0.565 | 0.486 | 0.270 | 0.644 | 0.491 | |
| 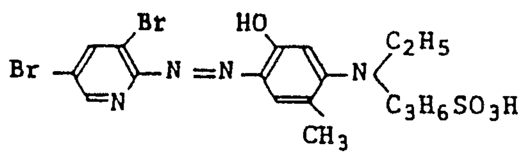 | No | 0.459 | 0.596 | 0.080 | 0.433 | 0.469 | |
| | Brij-35 | 0.422 | 0.569 | 0.021 | 0.320 | 0.424 | |
| | SLS | 0.452 | 0.463 | 0.035 | 0.407 | 0.428 | |
| | Sundet ENM | 0.343 | 0.516 | 0.115 | 0.381 | 0.408 | |

Reference Example 1

Reagents:

(1) Coloring reagent solution:

0.05 mmol of 5-Br-PAPS{2-(5-bromo-2-pyridylazo)-5-(N-propyl-N-sulfopropylamino)phenol sodium salt}, 33 g of sodium citrate, 1 g of salicylaldoxime and 0.03 mol of borax were dissolved in water to give 1 liter in a total volume, and the pH ws adjusted to 9.5 with hydrochloric acid.

(2) Sample solution A:
Same as Example 1

(3) Sample solution B:
Same as Example 1

(4) Sample solution C:
Same as Example 1

(5) Sample solution D:
Same as Example 1

(6) Sample solution E:
Same as Example 1

Test Method:

3 ml of the coloring reagent solution was added to 20 µl of each of Sample solutions A—E, which was left at room temperature for 20 minutes. Thereafter, absorbance was measured at 555 nm with reference to a reagent blank as control.

In Table 2 are shown absorbances of the respective metals and relative values of the metals when the coloring degree of zinc is taken as 100.

Table 2

| | Zn | Fe | Cu | Ni | Co |
|---|---|---|---|---|---|
| Absorbance | 0.726 | 0.091 | 0.027 | 0.832 | 0.461 |
| Relative value when the Coloring degree of Zinc is taken as 100 | 100 | 13 | 4 | 115 | 63 |

Example 2

Reagents:

(1) Coloring reagent solution:

0.05 mmol of 5Br-PAPS, 33 g of sodium citrate, 1 g of salicylaldoxime, 0.03 mol of borax, and the following surface active agent were dissolved in water to give 1 liter in total volume, and the pH was adjusted to 9.5 with hydrochloric acid.

As the surface active agent, Triton X—100, Brij-35, Emulgen 120, Tween 20, Tween 80, Softanol 90, Emusol 3130, Anhytol 24B, Cotamine 24P, Cotamine 86P, Levenol WX, sodium laurylsulfate, and Demol N respectively were singly used in an amount of 1.6%. Alternatively, Brij-35 and Triton X—100 were used together each at an amount of 1%, or sodium laurylsulfate and Demol N were used together each at an amount of 1%. (In the above, the respective percentages of the ingredients are expressed with respect to the weight of the whole coloring reagent solution.)

(2) Sample solution A:
Same as Example 1

(3) Sample solution B:
Same as Example 1

(4) Sample solution C:
Same as Example 1

(5) Sample solution D:
Same as Example 1

(6) Sample solution E:
Same as Example 1

Test Method:

3 ml of each of the coloring reagent solutions containing the respective surface active agents was added to 20 µl of Sample solutions A—E, which was left at room temperature for 20 minutes. Thereafter, absorption was measured at 555 nm with reference to a regent blank as control.

In Table 3 are shown the coloring percentages of the respective metals with respect to those in Reference Example 1, and the relative values of the metals when the coloring degree of zinc was taken as 100.

Table 3

| Surface active agent | | Coloring percentage | | | | |
|---|---|---|---|---|---|---|
| Classification | Trade name | Zn 5mg/dl | Fe 10mg/dl | Cu 5mg/dl | Ni 5mg/dl | Co 5mg/dl |
| Nonionic type | Triton X -100 | 109 % | 36 % | 93 % | 47 % | 63 % |
| | | 100 | 4 | 3 | 50 | 36 |
| | Brij-35 | 107 % | 13 % | 7 % | 58 % | 36 % |
| | | 100 | 2 | 0 | 62 | 21 |
| | Emulgen 120 | 94 % | 24 % | 39 % | 40 % | 72 % |
| | | 100 | 3 | 2 | 49 | 48 |
| | Tween 20 | 125 % | 76 % | 31 % | 57 % | 63 % |
| | | 100 | 8 | 1 | 52 | 32 |
| | Tween 80 | 105 % | 10 % | 41 % | 61 % | 60 % |
| | | 100 | 1 | 2 | 67 | 36 |
| | Softanol 90 | 101 % | 23 % | 70 % | 51 % | 62 % |
| | | 100 | 3 | 3 | 50 | 39 |
| | Emusol 3130 | 93 % | 19 % | 0 % | 72 % | 59 % |
| | | 100 | 3 | 0 | 89 | 40 |
| | Brij-35 Triton X-100 | 109 % | 25 % | 64 % | 45 % | 35 % |
| | | 100 | 3 | 2 | 48 | 20 |
| Amphoteric type | Anhytol 24B | 81 % | 6 % | 0 % | 42 % | 60 % |
| | | 100 | 1 | 0 | 60 | 47 |
| Cationic type | Cotamine 24P | 84 % | 21 % | 17 % | 39 % | 55 % |
| | | 100 | 3 | 1 | 53 | 41 |
| | Cotamine 86P | 67 % | 19 % | 0 % | 27 % | 50 % |
| | | 100 | 4 | 0 | 46 | 47 |
| Anionic type | Levenol WX | 101 % | 9 % | 51 % | 68 % | 63 % |
| | | 100 | 1 | 2 | 77 | 39 |
| | Sodium lauryl sulfate | 102 % | 27 % | 0 % | 54 % | 58 % |
| | | 100 | 3 | 0 | 61 | 36 |
| | Demol N | 88 % | 12 % | 24 % | 31 % | 35 % |
| | | 100 | 2 | 1 | 41 | 25 |
| | Sodium lauryl sulfate Demol N | 102 % | 15 % | 7 % | 45 % | 40 % |
| | | 100 | 2 | 1 | 51 | 25 |

(In the upper lines of the column of "Coloring percentage" are given the coloring percentages of the respective metals with respect to the coloring-percentages in Reference Example 1, whereas in the lower lines are given the relative values of the metals when the coloring degree of zinc is taken as 100).

As obvious from Tables 2 and 3, the specificity to zinc is notably enhanced by the addition of the surface active agents. In particular, the method of the present invention is excellent in reducing the coloring of iron and copper.

For instance, even when citric acid and salicylaldoxime are coexistent, the coloring degree of iron is 13 provided that iron is added in an amount of twice by weight as large as that of each of the other metal, and that of copper is 4, while the coloring degree of zinc is 100. On the contrary, when Brij-35, Emulgen 120, Tween 80, Emusol 3130, sodium lauryl sulfate (SLS), Levenol WX, Demol N, Cotamine 24P, or Anhytrol 24B was added in an amount of 1.6%, the coloring degree of iron is 1—3, and that of copper is 0—2, while the coloring degree of zinc is 100. Thus, the specificity to zinc is overwhelmingly enhanced. Since no practical problems rise in the clinically chemical analysis when the coloring of iron and copper is inhibited, this is an extremely important effect.

## Example 3

Reagents:

(1) Coloring reagent solution:

0.05 mmol of 5Br-PAPS, 33 g of sodium citrate, 1 g of salicylaldoxime, Alanon ACE in an amount of 1.6% or Alanon-ACE in an amount of 0.7% and Brij-35 in an amount of 1% as surface active agent, and 0.03 mol of borax were dissolved in water to give 1 liter in total volume, and the pH was adjusted to 9.5 with bydrochloric acid. (In the above, the respective percentages of the ingredients are expressed with respect to the weight of the whole coloring reagent solution.)

(2) Sample solution A:
Same as Example 1

(3) Sample solution B:
Same as Example 1

(4) Sample solution C:
Same as Example 1

(5) Sample solution D:
Same as Example 1

(6) Sample solution E:
Same as Example 1

Test method:

3 ml of each of the coloring reagent solutions containing the respective surface active agents was added to 20 µl of every one of Sample solutions A—E, which was left at room temperature for 20 minutes. Thereafter, absorbance was measured at 555 nm with reference to a reagent blank.

In Table 4 are shown the coloring percentages of the respective metals with respect to those in Reference Example 1, and the relative values of the metals when the coloring degree of zinc is taken as 100.

Table 4

| Surface active agent | | Coloring percentage | | | | |
|---|---|---|---|---|---|---|
| Anionic type | Nonionic type | Zn | Fe | Cu | Ni | Co |
| Alanon ACE | ——— | 1% | 9% | 121% | 57% | 8% |
| | | - | - | - | - | - |
| Alanon ACE (0.7%) | Brij-35 (1 %) | 90% | 15% | 100% | 18% | 0% |
| | | 100 | 2 | 4 | 23 | 0 |

(In the upper lines of the column "Coloring percentage" are shown the coloring percentages of the respective metals with respect the the coloring percentages in Reference Example 1, whereas in the lower lines are given the relative values of the respective metals when the coloring degree of zinc is taken as 100).

As obvious from Table 4, the coloring percentage of zinc is 0—1% when the anionic surface active agent was used singly, when the nonionic surface active agent is coexistent, zinc is removed form masking and cobalt is further masked.

As explained in the foregoing, when the surface active agents are appropriately selected and used in combination noting an interfering metal which is anticipated to be present in a sample, zinc can be quantitatively measured with accuracy.

## Example 4

Reagents:

(1) Coloring reagent solution:

0.05 ml of 5Br-PAPS, 33 g sodium citrate, 1 g of salicylaldoxime, 0.03 mol of borax, and 10 g of Brij-35 were dissolved in water to give 1 liter in total volume, and the pH was adjusted to 9.5 with hydrochloric acid.

(2) Sample solution:

Sample solution presumed to contain about 100 μg/dl of zinc

Measuring method:

3 ml of the coloring reagent solution was added to 1 ml of the sample solution, which was left at room temperature for 5 minutes. Thereafter, absorbance was measured at 555 nm with reference to a reagent blank as control to determine the concentration of zinc.

As obvious from Tables 2 and 3, the influence of iron is lowered to about one sixth at this case as compared with the case where no Brij-35 was added.

## Example 5

Reagents:

(1) Coloring reagent solution:

0.05 mmol of 5Br-PAPS, 33 g of sodium citrate, 1 g of salicylaldoxime, 20 g of Brij-35, and 20 g of Triton X—100 were dissolved in 0.05 M carbonate buffer solution to give 1 liter in total volume, and the pH was adjusted to 9.5 with a hydrochloric acid.

(2) Sample solution:

Serum

(3) EDTA·tetrasodium salt solution:

1 M EDTA·tetrasodium salt solution was prepared.

Measuring method:

2.5 ml of the coloring reagent solution was added to 200 μl of the sample solution, which was left at room temperature for 5 minutes. Thereafter, absorbance was measured as 555 nm. Then, a drop of the EDTA·tetrasodium salt solution was added for decoloring the solution, which was allowed to be left for 10 minutes. Subsequently, a sample blank was obtained by measuring absorbance at 555 nm through using the solution added with EDTA as control. The concentration of zinc was determined by subtracting the absorbance of the sample blank from that of the sample.

At this time, as obvious from Table 3, substantially no influence was observed even when iron or copper was present.

## Example 6

Reagents:

(1) Coloring reagent solution:

0.05 mmol of 5 Br-PAPS, 33 g of sodium citrate, 1 g of salicylaldoxime, 16 g of Emusol 3130 and 0.03 mol of borax were dissolved in water to give 1 liter, and the pH was adjusted to 9.5 with hydrochloric acid.

(2) Sample solution:

Same as Example 4

Measuring method:

3 ml of the coloring reagent solution was added to 1 ml of the sample solution, which was left at room temperature for 5 minutes. Thereafter, the absorbance was measured at 555 nm with reference to a reagent blank as control, and the concentration of zinc was determined therefrom.

At this time, as obvious from Table 3, substantially no influence was observed even when iron or copper was present.

Example 7

Reagents:

(1) Coloring reagent solution:

0.05 mmol of 5Br-PAPS, 1 g of salicylaldoxime, 7 g of Alanon ACE, 10 g of Brij-35, and 0.03 mol of borax were dissolved in water to give 1 liter in total volume, and the pH was adjusted to 9.5 with hydrochloric acid.

(2) Sample solution:

Same as Example 4

Measuring method:

3 ml of the coloring reagent was added to 1.0 ml of the sample solution, which was left at room temperature for 5 minutes. Thereafter, absorbance was measured at 555 nm with reference to a reagent blank as control, and the concentration of zinc was determined therefrom.

At this time, as obvious from Table 4, no interference was seen even when cobalt was coexistent.

Example 8

Reagents:

(1) Coloring reagent solution:

0.05 mmol of 5Br-PAPS, 33 g of sodium citrate, 1 g of salicylaldoxime, 16 g of Levenol WX and 0.03 mol of borax were dissolved in water to give 1 liter in total volume, and the pH was adjusted to 9.5 with hydrochloric acid.

(2) Sample solution:

Same as Example 4

Measuring method:

3 ml of the coloring reagent solution was added to 1 ml of the sample solution, which was left at room temperature for 5 minutes. Thereafter, absorbance was measured at 555 nm with reference to a reagent blank as control, and the concentration of zinc was determined therefrom.

At this time, as obvious from Table 3, substantially no infleunce was observed even when iron or copper was present.

Example 9

Reagent:

(1) Coloring reagent solution:

0.05 mmol of 5 Br-PAPS, 33 g of sodium citrate, 1 g of salicylaldoxime, 10 g of sodium laurylsulfate, 10 g of Demol N, and 0.03 mol of borax was dissolved in water to give 1 liter in total volume, and the pH was adjusted to 9.5 with hydrochloric acid.

(2) Sample solution:

Same as Example 4

Measuring method:

3 ml of the coloring reagent solution was added to 1 ml of the sample solution, which was left at room temperature for 5 minutes. Thereafter, absorbance was measured at 555 nm with reference to a reagent blank as control, and the concentration of zinc was determined therefrom.

At that time, as obvious from Table 3, substantially no interference in the measurement was observed even when iron or copper was present.

Example 10

Reagents:

(1) Coloring reagent solution:

0.05 mM of 5 Br-PAPS, 33 g of sodium citrate, 1 g of salicylaldoxime, 1.0 g of dimethylglyoxime, and the following surface active agents were dissolved in 1 liter of 0.05 M carbonate buffer solution of pH 9.5.

As the surface active agent, 40 g of Triton X—100 or 40 g of Brij-35 was singly used. Alternatively, 40 g of Triton X—100 and 40 g of Brij-35 were used in combination or no surface active agent was added.

(2) Sample solution A:

14 mg (5 ml when calculated as zinc) of zinc acetate was dissolved into water to be 100 ml in a total volume.

(3) Sample solution B:

25 mg (5 mg when calculated as iron) of ammonium iron (II) sulfate was dissolved into 0.1 N—HCl to be 100 ml in a total volume.

(4) Sample solution C:
24 mg (5 mg when calculated as iron) of ammonium iron (III) sulfate was dissolved into 0.1 N—HCl to be 100 ml in a total volume.

(5) Sample solution D:
18 mg (5 mg when calculated as copper) of copper sulfate was dissolved into water to be 100 ml in a total volume.

(6) Sample solution E:
11 mg (5 mg when calculated as nickel) of nickel chloride was dissolved into water to be 100 ml in a total volume.

(7) Sample solution F:
11 mg (5 mg when calculated as cobalt) of cobalt chloride was dissolved into water to be 100 ml in a total volume.

Experimental Method:
3 ml of the coloring reagent solution containing the surface active agent or no surface active agent was added to 20 μl of each of Sample solutions A—F, which was left at room temperature for 20 minutes. Thereafter, absorbance was measured at 555 nm with reference to a reagent blank as control. Results as shown in Table 5.

Table 5

| Surface active agent | Coloring degree | | | | | |
|---|---|---|---|---|---|---|
| | Zn | FeII | FeIII | Cu | Ni | Co |
| No addition | 0.732 | 0.111 | 0.097 | 0.015 | 0.476 | 0.009 |
| | 100.0 | 15.2 | 13.3 | 2.0 | 65.0 | 1.2 |
| Brij-35 | 0.763 | 0.014 | 0.006 | 0.001 | 0.026 | 0.001 |
| | 100.0 | 1.8 | 0.7 | 0.1 | 3.4 | 0.1 |
| Triton X-100 | 0.759 | 0.010 | 0.002 | 0.003 | 0.021 | 0.003 |
| | 100.0 | 1.3 | 0.3 | 0.4 | 2.8 | 0.4 |
| Brij-35 Triton X-100 | 0.762 | 0.004 | 0.000 | 0.000 | 0.008 | 0.001 |
| | 100.0 | 0.5 | 0.0 | 0.0 | 1.0 | 0.1 |

(The above lines in the column "Coloring degree" give the absorbances of the respective metals, whereas the lower lines give the relative values of the metals when the coloring degree of zinc is taken as 100).

Further, in Table 6 are shown results on the coloring degrees of the respective metals similarly measured when neither surface active agent nor the masking agent according to the present invention were added and results on the coloring degrees of the respective metals similarly measured when Brij-35, and Triton X—100 were used in combination without the masking agent of the present invention (dimethylglyoxime). At this time, 48 mg (10 mg when calculated as iron) of ammonium iron (III) sulfate which was dissolved in 0.1 N—HCl to be 100 ml in a total volume was used instead of Sample solution B or Sample solution C.

Table 6

| | Coloring degree | | | | |
|---|---|---|---|---|---|
| | Zn | FeIII | Cu | Ni | Co |
| Neither surface active agent nor dimethylglyoxime added | 0.726 | 0.091 | 0.027 | 0.832 | 0.461 |
| | 100.0 | 13 | 4 | 115 | 63 |
| No dimethylglyoxime added Surface active agent added | 0.791 | 0.023 | 0.017 | 0.374 | 0.161 |
| | 100.0 | 2.9 | 2.1 | 47.3 | 20.4 |

(The upper lines in the column "Coloring degree" show the absorbances of the respective metals, whereas the lower lines show the relative values of the metals when the coloring degree of zinc is taken as 100).

As obvious from Tables 5 and 6, the quantitative measurement of zinc can be accurately performed by appropriately selecting and combiningly using dimethylglyoxime, the conventionally used masking agent and the surface active agents.

Example 11

Reagents:

(1) Coloring reagent solution:

0.05 mmol of 5Br-PAPS, 33 g of sodium citrate, 1 g of salicyladoxime, 1 g of dimethylglyoxime, 40 g of Brij-35 and 40 g of Triton X—100 were dissolved in 0.05 M carbonate buffer solution to give 1 liter in total volume, and the pH was adjusted to 9.5 with hydrochloric acid.

(2) Sample solution

Serum

(3) EDTA·tetrasodium salt solution.

1M EDTA·tetrasodium salt solution was prepared.

Measuring method:

2.5 ml of the coloring reagent solution was added to 200 µl of the sample solution, which was left at room temperature for 5 minutes. Thereafter, absorbance was measured at 555 nm with reference to a reagent blank as control. A drop of 1 M EDTA solution was added to this coloring reagent solution, which was allowed to be left for 5 minutes after stirring. Then, absorbance was measured again at 555 nm with reference to a reagent blank to which EDTA was added as control, and the concentration of zinc was determined by substracting a sample blank therefrom.

Results are shown in Table 7 while being compared with those according to the atomic absorption method.

Table 7

| No. | Method of invention | Atomic absorption method | No. | Method of invention | Atomic absorption method |
|---|---|---|---|---|---|
| | (µg/dl) | (µg/dl) | | (µg/dl) | (µg/dl) |
| 1 | 108 | 106 | 22 | 97 | 100 |
| 2 | 86 | 94 | 23 | 81 | 85 |
| 3 | 63 | 63 | 24 | 85 | 88 |
| 4 | 64 | 63 | 25 | 75 | 75 |
| 5 | 75 | 75 | 26 | 112 | 106 |
| 6 | 66 | 56 | 27 | 44 | 38 |
| 7 | 106 | 106 | 28 | 84 | 81 |
| 8 | 80 | 81 | 29 | 90 | 85 |
| 9 | 76 | 75 | 30 | 80 | 75 |
| 10 | 89 | 88 | 31 | 86 | 75 |
| 11 | 88 | 85 | 32 | 104 | 113 |
| 12 | 93 | 94 | 33 | 63 | 63 |
| 13 | 77 | 75 | 34 | 95 | 94 |
| 14 | 106 | 106 | 35 | 74 | 75 |
| 15 | 91 | 94 | 36 | 80 | 81 |
| 16 | 63 | 63 | 37 | 108 | 106 |
| 17 | 77 | 85 | 38 | 75 | 63 |
| 18 | 61 | 63 | 39 | 97 | 94 |
| 19 | 89 | 88 | 40 | 78 | 81 |
| 20 | 83 | 82 | | | |
| 21 | 78 | 75 | average | 83.175 | 82.363 |

As obvious from Table 7, the method according to the present invention and the atomic absorption method are well in conformity with each other at a relation coefficient of 0.962.

## Claims

1. A method for colorimetrically measuring zinc using an aqueous solution of a 2-pyridylazoaminophenol derivative represented by the general formula (I):

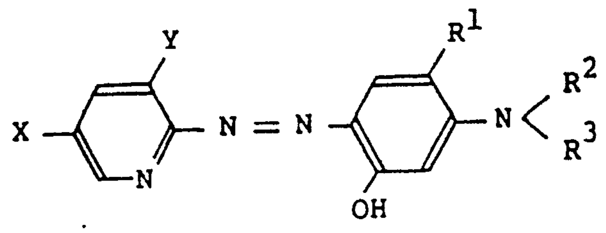

(I)

wherein X and Y represents a halogen atom or a hydrogen atom, $R^1$ represents a hydrogen atom or a $C_{1-4}$-alkyl group, and $R^2$ and $R^3$ represent a hydrogen atom, a $C_{1-4}$-alkyl group, $-(CH_2)_nSO_3H$ in which n is an integer of 1—4, or

$$-(CH_2)_l-\underset{}{\overset{OH}{\overset{|}{C}H}}-(CH_2)_m-SO_3H$$

in which l is an integer of 0—4 and m is an integer of 0—4, provided that at least one of $R^2$ and $R^3$ is $-(CH_2)_nSO_3H$ or

$$-(CH_2)_l-\underset{\underset{OH}{|}}{CH}-(CH_2)_m.SO_3H$$

or a salt thereof as a coloring reagent, characterized in that one or more kinds of surface active agents are used as a masking agent for Fe, Cu, Co and Ni.

2. A colorimetric measuring method according to claim 1, wherein the surface active agent is used in combination with and in addition to the conventional masking agents for Fe, Cu, Co, and Ni.

3. A colorimetric measuring method according to claims 1 and 2, wherein a nonionic surface active agent is used as the surface active agent in the method of colorimetrically measuring zinc by using a 2-pyridylazoaminophenol derivative represented by the general formula (I) or the salt thereof as the coloring reagent.

4. A colorimetric measuring method according to claims 1 and 2, wherein a cathionic surface active agent is used as the surface active agent in the method of colorimetrically measuring zinc by using a 2-pyridylazoaminophenol derivative represented by the general formula (I) or the salt thereof as the coloring reagent.

5. A colorimetric measuring method according to claims 1 and 2, wherein an amphoteric surface active agent is used as the surface active agent in the method of colorimetrically measuring zinc by using a 2-pyridylazoaminophenol derivative represented by the general formula (I) or the salt thereof as the coloring reagent.

6. A colorimetric measuring method according to claims 1 and 2 wherin an anionic surface active agent and a nonionic surface active agent are used in combination as the surface active agent in the method of colorimetrically measuring zinc by using a 2-pyridylazoaminopohenol derivative represented by the general formula (I) or the salt thereof as the coloring regent.

7. A method for colorimetrically measuring zinc by using as a coloring reagent a 2-pyridylazoaminophenol deritative represented by the following general formula (I):

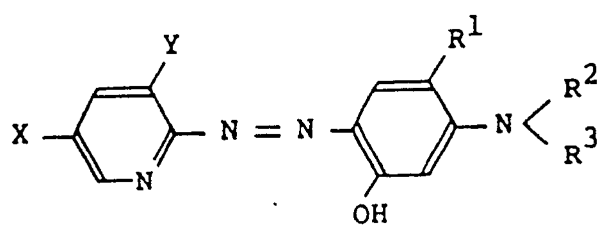

(I)

as defined in claim 1 and using one or more kinds of surface active agent in combination therewith, wherein dimethylglyoxime is used as a masking agent of nickel and/or cobalt.

**Patentansprüche**

1. Eine Methode zum kolorimetrischen Messen von Zink unter Verwendung einer wäßrigen Lösung eines 2-Pyridylazoaminophenolderivats, dargestellt durch die folgende allgemeine Formel(I), oder eines Salzes davon als Färbereagens:

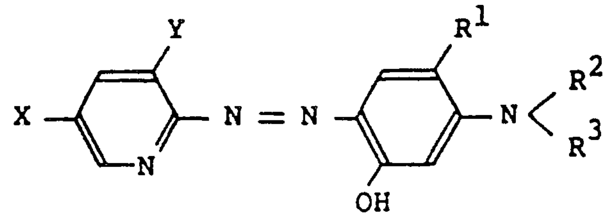

(I)

in welcher X und Y eine Halogenatom oder ein Wasserstoffatom bedeuten, $R^1$ ein Wasserstoffatom oder eine $C_{1-4}$-Alkylgruppe bedeutet und $R^2$ und $R^3$ ein Wasserstoffatom, eine $C_{1-4}$-Alkylgruppe, $-(CH_2)_n-SO_3H$, wobei n eine gerade Zahl von 1 bis 4 ist, oder

$$-(CH_2)_l-\underset{}{\overset{OH}{\overset{|}{CH}}}-(CH_2)_m-SO_3H$$

bedeuten, wobei l eine ganze Zahl von 0 bis 4 ist und m eine ganze Zahl von 0 bis 4 ist, mit der Maßgabe, daß mindestens eine der Gruppen $R^2$ und $R^3$ $-(CH_2)_n-SO_3H$ oder

$$-(CH_2)_l-\underset{\underset{OH}{|}}{CH}-(CH_2)_m.SO_3H$$

darstellt, dadurch gekennzeichnet, daß eine oder mehrere Arten von oberflächenaktiven Mitteln als Maskierungsmittel für Fe, Cu, Co und Ni eingesetzt werden.

2. Eine Methode zum kolorimetrischen Messen nach Anspruch 1, bei welcher das oberflächenaktive Mittel in Verbindung mit und zusätzlich zu den herkömmlichen Maskierungsmitteln für Fe, Cu, Co and Ni eingesetzt wird.

3. Eine Methode zum kolorimetrischen Messen nach den Ansprüchen 1 und 2, in welcher ein nichtionisches oberflächenaktives Mittel als das oberflächenaktive Mittel bei der Methode zum kolorimetrischen Messen von Zink unter Verwendung eines 2-Pyridylazoaminophenolderivats, dargestellt durch die allgemeine Formel (I), oder eines Salzes davon alks Färbereagens eingesetzt wird.

4. Eine Methode zum kolorimetrischen Messen nach den Ansprüchen 1 und 2, in welcher ein kationisches oberflächenaktives Mittel als das oberflächenaktive Mittel beim Verfahren zum kolorimetrischen Messen von Zink unter Versendung eines 2-Pyridylazoaminophenolderivats, dargestellt durch die allgemeine Formel (I), oder eines Salzes davon als Färbereagens eingesetzt wird.

5. Eine Methode zum kolorimetrischen Messen nach den Ansprüchen 1 und 2, in welcher ein amphoteres oberflächenaktives Mittel als das oberflächenaktive Mittel beim Verfahren zum kolorimetrischen Messen von Zink unter Verewendung eines 2-Pyridylazoaminophenolderivates, dargestellt durch die allgemeine Formel (I), oder eines Salzes davon alks Färbereagens eingesetzt wird.

6. Eine Methode zum kolorimetrischen Messen nach den Ansprüchen 1 und 2, in welcher ein anionisches oberflächenaktives Mittel und ein nichtionisches oberflächenaktives Mittel zusammen als das oberflächenaktive Mittel im Verfahren zum kolorimetrischen Messen von Zink unter Verwendung eines 2-Pyridylazoaminophenolderivats, dargestellt durch die allgemeine Formel (I), oder des Salzes davon als das Färbereagens eingesetzt werden.

7. Eine Methode zum kolorimetrischen Messen von Zink unter Verwendung eines 2-Pyridylazoaminophenolderivats als Färbereagens dargestellt durch die foglende allgemeine Formel (I):

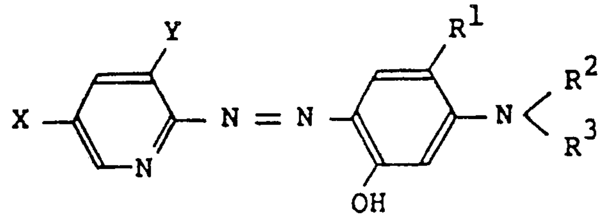

(I)

wie in Anspruch 1 definiert und unter Verwendung einer oder mehrerer Arten von oderflächenaktiven Mitteln in Kombination damit, wobei Dimethylglyoxim als ein Maskierungsmittel für Nickel und/oder Kobalt verwendet wird.

## Revendications

1. Procédé de mesure ou détermination colorimétrique du zinc, par utilisation d'une solution aqueuse d'un dérivé de 2-pyridylazoaminophénol représenté par la formule générale (I) suivant:

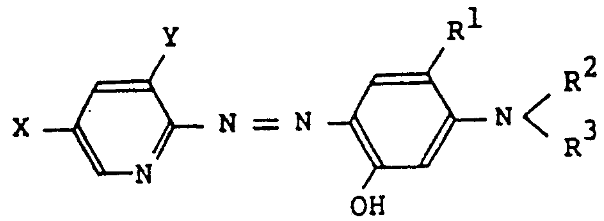

(I)

[dans laquelle X et Y représentent chacun un atome d'halogène ou un atome d'hydrogène; $R^1$ représente un atome d'hydrogène ou un groupe alkyl en $C_1$ à $C_4$, et $R^2$ et $R^3$ représentent chacun un atome d'hydrogène, un groupe alkyle en $C_1$ à $C_4$, $-(CH_2)_n-SO_3H$ où n est un nombre entier valant 1 à 4, ou

$$-(CH_2)_l-\underset{\displaystyle OH}{\underset{|}{CH}}-(CH_2)_m-SO_3H,$$

où l est un nombre entier valant 0 à 4, et m est un nombre entier valant 0 à 4, à la conditon qu'au moins l'un des symboles $R^2$ et $R^3$ représente $-(CH_2)_n-SO_3H$ ou

$$-(CH_2)_l-\underset{\displaystyle OH}{\underset{|}{CH}}-(CH_2)_m.SO_3H],$$

ou un de ses sels comme réactif de coloration, procédé caractérisé en ce qu'on utilise un ou plusieurs genres d'agents tensio-actifs comme agent de masquage de Fe, de Cu, de Co et de Ni.

2. Procédé de mesure ou détermination colorimétrique selon la revendication 1, dans lequel on utilise l'agent tensio-actif en combinaison avec, et en plus, des agents classiques de masquage de Fe, Cu, Co et Ni.

3. Procédé de mesure ou détermination colorimétrique selon la revendications 1 et 2, dans lequel on utilise un agent tensio-actif non ionique comme agent tensio-actif dans le procédé de mesure ou détermination colorimétrique du zinc, en utilisant, comme réactif de coloration, un dérivé de 2-pyridylazoaminophénol représenté par la formule générale (I) ou un de ses sels.

4. Procédé de mesure ou détermination colorimétrique selon les revendications 1 et 2, dans lequel on utilise un agent tensio-actif catoinique comme agent tensio-actif dans le procédé de mesure ou de détermination colorimétrique du zinc, en utilisant comme réactif de coloration un dérivé de 2-pyridylazoaminophénol, représenté par la formule générale (I), ou son sel.

5. Procédé de mesure ou détermination colorimétrique selon les revendications 1 et 2, dans lequel on utilise un agent tensio-actif amphotère comme agent tensio-actif dans le procédé de détermination ou mesure colorimétrique du zinc, en utilisant comme réactif de coloration un dérivé de 2-pyridylazoaminophénol représenté par la formule générale (I) ou un de ses sels.

6. Procédé de détermination ou mesure colorimétrique selon les revendications 1 et 2, dans lequel on utilise un agent tensio-actif anionique et un agent tensio-actif non ionique, en combinaison, pour constituer l'agent tensio-actif dans le procédé de mesure ou détermination colorimétrique du zinc, en utilisant, comme réactif de coloration, un dérivé de 2-pyridylazoaminophénol, représenté par la formule générale (I) ou un de ses sels.

7. Procédé pour mesurer ou déterminer colorimétriquement le zinc, en utilisant comme réactif de coloration un dérivé de 2-pyridylazoaminophénol représenté par la formule générale (I) suivante:

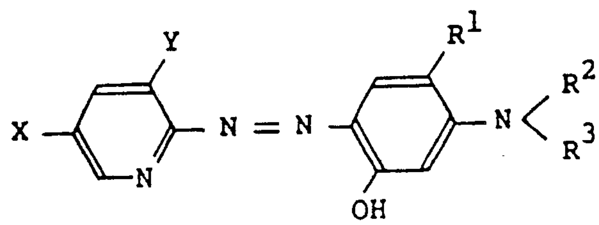

(I)

selon la définition donnée à la revendication 1, et en utilisant un ou plusieurs genres d'agent tensio-actif en combinaison avec ce réactif de coloration, procédé dans lequel on utilise la diméthylglyoxime comme agent de masquage du nickel et/ou du cobalt.